# EUROPEAN PATENT APPLICATION

(11) **EP 4 582 095 A1**
(43) Date of publication of application: **09.07.2025**
(21) Application number: 23860913.5
(22) Date of filing: 31.08.2023
(51) Int. Cl.: A61K 38/08, A61P 17/00, C07K 7/06

(54) **PEPTIDE FOR PROMOTING TYROSINASE ACTIVITY AND USE THEREOF**

(30) Priority: 31.08.2022 KR 20220109842
(71) Applicant: Hysensbio Co., Ltd, Gwacheon-si Gyeonggi-do 13814 (KR)
(72) Inventor: PARK, Jung Bum, Gunpo-si Gyeonggi-do 15806 (KR); PARK, Joo Hwang, Incheon 21986 (KR)
(74) Representative: Mewburn Ellis LLP
(86) International application number: PCT/KR2023/012998
(87) International publication number: WO 2024/049238

(57) **Abstract**

A composition for promoting tyrosinase activity according to the present invention includes a peptide consisting of the amino acid sequence of General Formula 1 below: K-Y-R1-R2-R3-R4-R5-R6-R7-R8 (General Formula 1). In General Formula 1, R1 is arginine (R), lysine (K), or glutamine (Q); R2 is arginine (R) or glutamine (Q); R3, R4, and R5 are each arginine (R) or lysine (K); R6 is asparagine (N) or serine (S); and R7 and R8 are each lysine (K) or tyrosine (Y). The composition according to the present invention may promote melanogenesis in melanocytes to prevent, improve or treat hypomelanosis.

## Description

### [Technical Field]

The present invention relates to a peptide promoting tyrosinase activity and a use thereof, and more particularly, to a peptide which promotes tyrosinase activity in melanocytes for preventing or treating hypomelanosis, and a use thereof.

### [Background Art]

Melanocytes are present in the epidermal layer of the skin. Melaine is a polymeric substance of phenols found widely in nature and is a complex of a black pigment and a protein. Regarding melanogenesis, melanin is synthesized in the organelles in melanocytes called melanosomes.

Melanocytes extend in a dendrite shape and are surrounded by keratinocytes. Melanocytes form melanosomes and deliver them to keratinocytes. Melanosomes, in which melanin pigment is accumulated, are arranged to ascend through the dendritic processes of melanocytes and surround keratinocytes that produce skin cells, thereby preventing the DNA of the keratinocytes from being destroyed by UV rays or the like.

Melanin is synthesized in melanocytes from an amino acid called tyrosine, which is converted to 3,4-dihydroxyphenylalanin (L-DOPA), and then L-DOPA is oxidized again to produce DOPA-quinone. DOPA-quinone is converted to pheomelanin via cysteinyldopa. However, under the condition of cysteine deficiency, DOPA-quinone is converted to dopachrome by a nonenzymatic reaction. Dopachrome is converted to eumelanin through two processes: an enzymatic process involving TRP1 and TRP2, and a non-enzymatic process. The melanin produced in this way is delivered to surrounding keratinocytes in the form of vesicles called melanosomes by the dendrites of the melanocytes and spread evenly across the epidermis.

Meanwhile, vitiligo is an acquired skin condition in which the skin loses its color due to a lack of melanocytes in the skin, causing white patches. The clinical presentation of vitiligo is the development of white patches. This is due to damaged melanocytes, but the exact cause of the damage is unclear. There are various hypotheses as to the cause, and genetic factors, autoimmunity, oxidative stress, or harmful chemicals are believed to be the cause.

Areas where human vitiligo often occurs are the face and extremities. Skin discoloration caused by vitiligo can affect one's appearance and can be a source of stress in interpersonal relationships, and in fact, it is known that stress resulting from vitiligo significantly increases the risk of mental illness. The worldwide prevalence of vitiligo is approximately 0.5 to 1%, and the prevalence rates for men and women are known to be almost the same. There are differences in the prevalence of vitiligo by gender, and it is known that the disease develops at an earlier age in women than in men.

The accumulation of melanin is promoted by light such as UV rays, so phototherapy using this principle has been used to treat vitiligo patients since the 1800s. Several studies have shown that phototherapy promotes the migration and proliferation of melanocytes in the epidermal layer of the skin, providing a favorable environment for melanocyte growth and suppressing autoimmunity. Phototherapy using UVB has side effects such as itching, a burning sensation, erythema, temporary hyperpigmentation, blistering, and dryness, and all phototherapies are known to cause premature photoaging, making it difficult to use as a long-term treatment method.

Vitiligo is known to be treated with corticosteroid therapy. Corticosteroid is a term that refers to all steroids that have anti-inflammatory effects. It shows better results when used together with the phototherapy described above. Corticosteroid therapy causes pigmentation and halts the progression of the disease. However, this therapy has side effects such as epidermal atrophy, steroid folliculitis, and telangiectasia, and when used as a systemic therapy, side effects such as insomnia, acne, menstrual disorders, and weight gain may occur. Due to concerns about these side effects, corticosteroid therapy requires periodic breaks.

Another treatment method is a surgical treatment, such as epidermal transplantation or cell transplantation, but it is not yet widely used, cannot treat a large area, and has side effects such as scarring, infection, and graft failure. When vitiligo also occurs in the transplanted cells, this treatment method can make vitiligo worse.

### [Disclosure]

### [Technical Problem]

The present invention is directed to providing a peptide which promotes the formation of melanosomes by promoting tyrosinase activity in melanocytes, and a use thereof.

The present invention is also directed to providing a peptide which can prevent hypomelanosis, which causes depigmented patches on the skin, by regulating tyrosinase activity in melanocytes, and a use thereof.

The present invention is also directed to providing a peptide which can improve or treat hypomelanosis by promoting melanogenesis in melanocytes, and a use thereof.

The present invention is also directed to providing a peptide which promotes the process of oxidizing L-DOPA to DOPA-quinone during melanin synthesis in melanocytes, and a use thereof.

The objects of the present invention are not limited to those described above, and other objects which are not mentioned above can be more clearly understood by those of ordinary skill in the art to which the present invention belongs from the following description.

### [Technical Solution]

To solve the technical problems described above, one aspect of the present invention provides a composition for promoting tyrosinase activity, which includes a peptide consisting of the amino acid sequence of General Formula 1 below:

K-Y-R1-R2-R3-R4-R5-R6-R7-R8 (General Formula 1)

In General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are each lysine (K) or tyrosine (Y).

Another aspect of the present invention provides a composition for regulating melanin synthesis, which includes a peptide consisting of the amino acid sequence of General Formula 1.

Still another aspect of the present invention provides a composition for preventing, improving, or treating hypomelanosis, which includes a peptide consisting of the amino acid sequence of General Formula 1.

As long as it is capable of exhibiting an effect of promoting tyrosinase activity, a mutant peptide having a sequence differing by one or more amino acid residues from the amino acid sequence constituting the peptide of the present invention is also included in the scope of the peptides provided by the present invention.

Generally, amino acid exchanges in proteins and polypeptides, which do not alter the overall activity of a molecule, are known in the art. The most common exchanges are exchanges between amino acid residues, such as Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In addition, the peptide of the present invention may include a peptide which has increased structural stability of the peptide against heat or pH or an increased ability to promote melanosome synthesis due to a mutation or modification in the amino acid sequence.

For example, even when glutamine, which is an acidic amino acid located at position 3 of the peptide of SEQ ID NO: 1 provided by the present invention, is substituted by lysine or arginine, which are basic amino acids, the effect of the peptide provided by the present invention may still be exhibited; even when arginine, which is a basic amino acid located at position 4 or 5 of the peptide of SEQ ID NO: 1, is substituted by an acidic amino acid, glutamine, or a basic amino acid, lysine, the effect of the peptide provided by the present invention may still be exhibited; even when lysine, which is a basic amino acid located at position 6, 7, or 9 of the peptide of SEQ ID NO: 1, is substituted by a basic amino acid, arginine, or an aromatic amino acid, tyrosine, the effect of the peptide provided by the present invention may still be exhibited; even when asparagine, which is an acidic amino acid located at position 8 of the peptide of SEQ ID NO: 1, is substituted by a neutral amino acid, serine, the effect of the peptide provided by the present invention may still be exhibited; and even when tyrosine, which is an aromatic amino acid located at position 10 of the peptide of SEQ ID NO: 1, is substituted by a basic amino acid, lysine, the effect of the peptide provided by the present invention may still be exhibited.

Likewise, even when the acidic amino acids, basic amino acids, or aromatic amino acids constituting the peptide of the present invention are substituted with different acidic amino acids, basic amino acids, neutral amino acids, or aromatic amino acids, the effect of the peptide provided by the present invention may still be exhibited, so it is apparent that a variant peptide having a sequence that differs by one or more amino acid residues from the amino acid sequence constituting the peptide of the present invention is also included in the scope of the peptide provided by the present invention.

In addition, since the peptide of the present invention can exhibit the effect of the peptide provided by the present invention even when it has any amino acid added to its N- or C-terminus, it is included in the scope of the peptide provided by the present invention. As an example, the peptide of the present invention may have 1 to 300 amino acids added to its N- or C-terminus, as another example, have 1 to 100 amino acids added to its N- or C-terminus, or as still another example, have 1 to 24 amino acids added to its N- or C-terminus.

Yet another aspect of the present invention provides a polynucleotide which encodes the peptide.

The polynucleotide may be mutated by substitution, deletion, insertion, or a combination thereof of one or more bases. When a nucleotide sequence is produced by chemical synthesis, it may be synthesized using synthesis methods widely known in the art, for example, the method described in the literature (Engels and Uhlmann, Angew Chem IntEd Engl., 37:73-127, 1988), triester, phosphite, phosphoramidite and H-phosphate methods, PCR and other autoprimer methods, and an oligonucleotide synthesis method on a solid support. For example, a polynucleotide encoding the peptide of the present invention may include the base sequence of SEQ ID NO: 4.

Yet another aspect of the present invention provides an expression vector including the polynucleotide, a transformant including the expression vector, and a method of producing the peptide using the transformant.

The term "expression vector" used herein refers to a recombinant vector which can express a desired peptide in desired host cells, and refers to a gene construct including essential regulatory elements operably linked for the expression of a gene insert. The expression vector includes expression regulatory elements, such as a start codon, a termination codon, a promoter, and an operator, wherein the start codon and the termination codon are generally considered to be part of the nucleotide sequence encoding a polypeptide, and must be functional in a subject when the gene construct is inserted, and must be in frame with the coding sequence. The promoter of the vector may be constitutive or inducible.

The term "operably linked" used herein refers to a state in which a nucleic acid expression regulatory sequence and a nucleic acid sequence encoding a desired protein or RNA are functionally linked to perform a general function. For example, a promoter and a nucleic acid encoding a protein or RNA may be operably linked to affect the expression of the coding sequence. The operable linkage with the expression vector may be made using genetic recombination techniques well known in the art, and site-specific DNA cleavage and linkage can be done using enzymes generally known in the art.

In addition, the expression vector may include a signal sequence for releasing the peptide to promote the isolation of the peptide from a cell culture. Specific initiation signals may also be required for efficient translation of an inserted nucleic acid sequence. These signals include the ATG start codon and adjacent sequences. In some cases, an exogenous translational control signal, which may include the ATG start codon, must be provided. The exogenous translational control signal and the start codon may be from various natural and synthetic sources. Expression efficiency can be increased by the introduction of appropriate transcription- or translation-enhancing factors.

Moreover, the expression vector may further include a protein tag which can be optionally removed using endopeptidase to facilitate detection of the peptide.

The term "tag" used herein means a molecule that exhibits a quantifiable activity or property, and may be a fluorescent molecule which includes a chemical fluorescent material (fluoracer) such as fluorescein, or a polypeptide fluorescent material such as a fluorescent protein (GFP) or a related protein; or an epitope tag such as an Myc tag, a flag tag, a histidine tag, a leucin tag, an IgG tag, or a streptavidin tag. Particularly, when using an epitope tag, a peptide tag consisting of, preferably, 6 or more amino acid residues, and more preferably, 8 to 50 amino acid residues may be used.

In the present invention, the expression vector may include a nucleotide sequence encoding a peptide which provides the above-mentioned tyrosinase activity-promoting effect of the present invention. The vector used herein is not particularly limited as long as it can produce the peptide, and is preferably a plasmid, DNA, or phage DNA, more preferably, a commercially-developed plasmid (pUC18, pBAD, or pIDTSAMRT-AMP), an E. coli-derived plasmid (pYG601BR322, pBR325, pUC118, or pUC119), a Bacillus subtilis-derived plasmid (pUB110, or pTP5), an enzyme-derived plasmid (YEp13, YEp24, or YCp50), phage DNA (Charon4A, Charon21A, EMBL3, EMBL4, λgt10, λgt11, or λZAP), an animal virus vector (retrovirus, adenovirus, or vaccinia virus), or an insect virus vector (baculovirus). The expression vector shows a different protein expression level and modification depending on a host cell, so it is desirable to select and use the host cell most suitable for the purpose.

The transformant provided by the present invention may be produced by introducing the expression vector provided by the present invention into a host, and used to produce the peptide by expressing the polynucleotide included in the expression vector. The transformation may be performed by various methods, which are not particularly limited as long as the peptide can be produced. However, these methods may include a CaCl₂ precipitation method, a Hanahan method with higher efficiency by using dimethyl sulfoxide (DMSO) as a reducing material in the CaCl₂ precipitation method, electroporation, a calcium phosphate precipitation method, a protoplast fusion method, a stirring method using a silicon carbide fiber, an Agrobacterium-mediated transformation method, a transformation method using PEG, dextran sulfate, lipofectamine, and a drying/inhibition-mediated transformation method. In addition, the host used in the production of the transformant is also not particularly limited as long as it can produce the peptide, and the host may be bacterial cells such as E. coli, Streptomyces, or Salmonella typhimurium; yeast cells such as Saccharomyces cerevisiae or Schizosaccharomyces pombe; fungal cells such as Peachia pastoris; insect cells such as Drosophila or Spodoptera Sf9 cells; animal cells such as CHO, COS, NSO, 293, or Bow melanoma cells; or plant cells.

The transformant may also be used in a method for producing a peptide that provides the tyrosinase activity-promoting effect of the present invention. Specifically, the method for producing a peptide that provides the tyrosinase activity-promoting effect of the present invention may include (a) culturing the transformant to obtain a culture; and (b) recovering the peptide of the present invention from the culture.

The term "culture" used herein refers to a method of growing microorganisms under appropriately artificially controlled environmental conditions. In the present invention, the transformant may be cultured using a method widely known in the art. Specifically, the culturing method is not particularly limited as long as it can express and produce the peptide that provides the tyrosinase activity-promoting effect of the present invention, and the peptide may be continuously cultured in a batch process, or a fed batch or repeated fed batch process.

A medium used for culturing must meet the requirements of a specific strain in an appropriate manner while adjusting a temperature, pH, and the like under aerobic conditions in a normal medium containing appropriate carbon sources, nitrogen sources, amino acids, vitamins, or the like. Carbon sources that can be used herein include a mixed sugar of glucose and xylose as a main carbon source, as well as sugars and carbohydrates such as sucrose, lactose, fructose, maltose, starch, or cellulose, oils and fats such as soybean oil, sunflower oil, castor oil, and coconut oil, fatty acids such as palmitic acid, stearic acid, or linoleic acid, alcohols such as glycerol or ethanol, and organic acids such as acetic acid. These materials may be used separately or as a mixture. Nitrogen sources that can be used herein may include inorganic nitrogen sources such as ammonia, ammonium sulfate, ammonium chloride, ammonium acetate, ammonium phosphate, ammonium carbonate, and ammonium nitrate; amino acids such as glutamic acid, methionine, and glutamine; and organic nitrogen sources such as peptone, NZ-amine, a meat extract, a yeast extract, a malt extract, corn steep liquor, casein hydrolysate, fish or its decomposition product, defatted soybean cake or its decomposition product. These nitrogen sources may be used independently or in combination. The medium may contain potassium phosphate monobasic or potassium phosphate dibasic, and corresponding sodium-containing salts as a phosphorous source. Phosphorous sources that can be used herein include potassium dihydrogen phosphate, dipotassium hydrogen phosphate, or corresponding sodium-containing salts. In addition, as an organic compound, sodium chloride, calcium chloride, iron chloride, magnesium sulfate, iron sulfate, manganese sulfate, and calcium carbonate may be used. Finally, in addition to the above materials, essential growth materials such as amino acids and vitamins may be used.

In addition, precursors suitable for the culture medium may be used. The above-mentioned ingredients may be added to the culture in a batch, fed-batch or continuous manner during the culturing process, but are not particularly limited thereto. The pH of the culture may be adjusted by using basic compounds such as sodium hydroxide, potassium hydroxide, ammonia, or acid compounds such as phosphoric acid or sulfuric acid in an appropriate manner.

In addition, foaming may be suppressed by using an antifoaming agent such as fatty acid polyglycol ester. Oxygen or oxygen-containing gas (e.g., air) is injected into the culture to maintain an aerobic state. The temperature of the culture is usually 27 to 37 °C, preferably 30 to 35 °C. The culturing continues until the maximum production of the peptide is achieved. This purpose is usually achieved within 10 to 100 hours.

In addition, the step of recovering the peptide from the culture may be performed by a method known in the art. Specifically, the recovery method is not particularly limited as long as it can be used to recover the produced peptide, but preferably a method such as centrifugation, filtration, extraction, spraying, drying, evaporation, precipitation, crystallization, electrophoresis, fractionation (e.g., ammonium sulfate precipitation), and chromatography (e.g., ion exchange, affinity, hydrophobic, and size-exclusion chromatography) may be used.

The term "prevention" used herein refers to all actions of inhibiting or delaying the development of hypomelanosis by administering a pharmaceutical composition for preventing or treating hypomelanosis, including the peptide of the present invention.

The term "treatment" used herein refers to all actions that promote the synthesis of melanosomes by administering a pharmaceutical composition including the peptide of the present invention as an active ingredient to a subject in need of treatment of hypomelanosis, thereby performing the treatment of hypomelanosis.

The pharmaceutical composition of the present invention may be prepared in the form of a pharmaceutical composition for treating hypomelanosis, which further includes a suitable carrier (natural or non-natural carrier), excipient or diluent commonly used in the preparation of a pharmaceutical composition in addition to the peptide. Specifically, the pharmaceutical composition may be formulated in the form of a sterile injectable solution that can be administered to the area where hypomelanosis is induced according to a conventional method. In the present invention, a carrier, an excipient, and a diluent, which can be included in the pharmaceutical composition, may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, and collagen. When formulating, commonly used diluents or excipients such as a filler, a thickener, a binder, a wetting agent, a disintegrant, and a surfactant may be used. In particular, sterilized aqueous solutions, non-aqueous solvents, suspensions, emulsions, freeze-dried formulations, suppositories, or ointments (e.g., a pulp liner) may be included. As the non-aqueous solvent or suspension, propylene glycol, polyethylene glycol, a vegetable oil such as olive oil, or an injectable ester such as ethyl oleate may be used. As a suppository base, Witepsol, Tween 61, cacao butter, laurin butter, or glycerogelatin may be used.

The content of the peptide included in the pharmaceutical composition of the present invention may be, but is not particularly limited to, 0.0001 to 50 wt%, and preferably, 0.01 to 20 wt% based on the total weight of the final composition.

The pharmaceutical composition of the present invention may be administered at a pharmaceutically effective amount, and the term "pharmaceutically effective amount" used herein refers to an amount sufficient for treating or preventing a disease at a reasonable benefit/risk ratio applicable for medical treatment, and an effective dosage may be determined by factors including severity, drug activity, a patient's age, weight, health, sex, and drug sensitivity, the administration time, administration route and excretion rate of the composition of the present invention used, treatment period, and drugs used in combination with or concurrently used with the composition of the present invention, and other factors well known in the medical field. The pharmaceutical composition of the present invention may be administered alone or in combination with a known pharmaceutical composition for treating hypomelanosis. Considering all of the above factors, it is important to achieve the maximum effect with the minimum dose without side effects.

The dosage of the pharmaceutical composition of the present invention may be determined by those of ordinary skill in the art in consideration of the purpose of use, the severity of a disease, a patient's age, weight, gender, and medical history, or the type of a material used as an active ingredient. For example, the pharmaceutical composition of the present invention may be administered at approximately 0.1 ng to approximately 100 mg/kg, and preferably, approximately 1 ng to approximately 10 mg/kg per adult, and administration may be performed once a day or divided into several doses, but the present invention is not particularly limited thereto. The above dosage does not limit the scope of the present invention in any way.

Yet another aspect of the present invention provides a method of treating hypomelanosis, which includes administering the pharmaceutical composition to a subject with hypomelanosis in a pharmaceutically effective amount.

The term "vitiligo" used herein refers to an acquired depigmentation disorder in which white patches of various sizes and shapes appear on the skin due to the destruction of pigment cells.

The term "poliosis" used herein refers to a depigmented disorder in which melanin (or pigment) is reduced or disappears in the hair, eyebrows, and eyelashes.

The term "subject" used herein may include, without limitation, mammals including mice, livestock and the like, which require treatment for hypomelanosis, but may exclude humans from among the subjects with the disease.

The administration of the pharmaceutical composition for treating hypomelanosis of the present invention may be performed via a common route as long as the pharmaceutical composition of the present invention can reach target tissue. The pharmaceutical composition of the present invention is not particularly limited, but may be provided in any formulation suitable for topical application depending on the desired purpose. For example, the pharmaceutical composition of the present invention may be administered orally, transdermally, intravenously, intramuscularly, or subcutaneously. The pharmaceutical composition may be an injection, a topical solution, a suspension, an emulsion, a gel, a patch, or a spray, but the present invention is not limited thereto. The above-mentioned formulations may be easily manufactured according to a conventional method in the related art, and a surfactant, an excipient, a solubilizer, an emulsifying agent, a suspending agent, a salt or buffer for osmotic pressure control, a coloring agent, a flavoring agent, a stabilizer, an antiseptic, a preservative, or other commercially available adjuvants may be appropriately used.

Yet another aspect of the present invention provides a quasi-drug composition for preventing or improving hypomelanosis, which includes the peptide.

The term "improvement" used herein means any action that at least reduce parameters related to the condition being treated, for example, the severity of symptoms.

In the present invention, the improvement may be interpreted to mean all acts that improve or benefit the symptoms of hypomelanosis by promoting the synthesis of melanosomes by administering a pharmaceutical composition including the peptide of the present invention as an active ingredient to a subject in need of treatment of hypomelanosis.

The term "quasi-drug" used herein refers to products with milder effects than medicines among the products used for the purpose of diagnosing, treating, improving, alleviating, curing, or preventing human or animal diseases. For example, according to the Pharmaceutical Affairs Act, quasi-drugs are products other than those used for medicinal purposes, and include fiber and rubber products used for the treatment or prevention of human or animal diseases, products that have a mild or no direct effect on the human body and those similar thereto that are not an instrument or machine, and sterilizers and insecticides used to prevent infectious diseases.

According to the present invention, the type or formulation of the quasi-drug composition including the peptide is not particularly limited, but may be, for example, a disinfectant cleanser for skin or hair, a skin or hair cleaning product, soap, a shampoo, or an ointment for skin.

Yet another aspect of the present invention provides a health functional food composition for preventing or improving hypomelanosis, which includes the peptide.

The term "food" of the present invention includes meat, sausage, bread, chocolate, candy, snacks, confectioneries, pizza, ramen, other noodles, gum, dairy products including ice cream, various soups, beverages, tea, drinks, alcoholic beverages, vitamin complexes, health functional food, and health foods, and all types of food in the usual sense.

The health functional food is the same term as food for special health use (FoSHU), and means food with high medical and health effects processed to effectively display bioregulatory functions in addition to nutrition supply. Here, "function" means obtaining a useful effect for health purposes such as regulating nutrients or physiological functions for the structure and function of the human body. The food of the present invention may be manufactured by a method commonly used in the art, and during manufacturing, raw materials and ingredients commonly added in the art may be added. In addition, the formulation of the food may be manufactured without limitation as long as it is a formulation recognized as food. The food composition of the present invention may be manufactured in various formulations, and unlike general medicines, it has the advantage of not having side effects that may occur with long-term use of medicines because it uses food as a raw material, and it is easy to carry, so it can be consumed as a supplement for enhancing the effect of preventing or improving hypomelanosis.

The health food refers to food having an active health maintenance or enhancement effect, compared to general food, and health supplement food refers to food for health supplementation. In some cases, the terms health functional food, health food, and health supplement food may be used interchangeably.

Specifically, the health functional food is food manufactured by adding the peptide of the present invention to a food material such as a beverage, tea, spice, gum, and confectionery, or manufactured in the form of a capsule, powder, or suspension. Consuming the health function food means that it has a specific health effect, but unlike general medicines, the health function food is made from food, so it has the advantage of not having side effects that can occur with long-term use of medicines.

Since the food composition of the present invention can be consumed on a daily basis, it can be expected to be highly effective in preventing or improving hypomelanosis, and therefore, it can be used very usefully.

The food composition may further include a physiologically acceptable carrier, and the type of carrier is not particularly limited and may be any carrier conventionally used in the art.

In addition, the food composition may include additional ingredients commonly used in food compositions to improve smell, taste, and appearance. For example, the food composition of the present invention may include vitamins A, C, D, E, B1, B2, B6, and B12, niacin, biotin, folate, and pantothenic acid. In addition, the food composition of the present invention may include minerals such as zinc (Zn), iron (Fe), calcium (Ca), chromium (Cr), magnesium (Mg), manganese (Mn), and copper (Cu). In addition, the food composition of the present invention may include amino acids such as lysine, tryptophan, cysteine, and valine.

In addition, the food composition may also include food additives, for example, a preservative (e.g., potassium sorbate, sodium benzoate, salicylic acid, or sodium dehydroacetate), a disinfectant (e.g., bleaching powder, high-purity bleaching powder, or sodium hypochlorite), an antioxidant (e.g., butylated hydroxyanisole (BHA) or butylated hydroxy toluene (BHT)), a pigment (e.g., tar pigment), a colorant (e.g., sodium nitrite), a bleaching agent (e.g., sodium sulfite), a flavor enhancer (e.g., monosodium glutamate (MSG)), a sweetener (e.g., dulcin, cyclamate, saccharin, or sodium), a flavoring (e.g., vanillin or lactone), a leavening agent (e.g., alum or D-potassium hydrogen tartrate), a strengthener, an emulsifier, a thickener (a gelling agent), a coating agent, a gum base, an antifoaming agent, a solvent, and a conditioner. These additives may be selected according to the type of food and used in appropriate amounts.

The peptide of the present invention may be added as is or used in combination with another food or food component, and may be used appropriately according to a conventional method. The mixing amount of the active ingredient may be appropriately determined depending on its intended use (prevention, health, or a therapeutic method). Generally, when manufacturing a food or beverage, the food composition of the present invention may be added to the food or beverage in an amount of 50 parts by weight or less, specifically, 20 parts by weight or less. However, in the case of prolonged consumption for health and hygiene, the peptide of the present invention may be included at a content below the above range, and since there are no safety issues, the active ingredient may also be used in amounts higher than the above range.

As an example of the food composition of the present invention, a health beverage composition may be used, and in this case, like common drinks, it may contain additional ingredients such as various flavorings or natural carbohydrates. The natural carbohydrates may be monosaccharides such as glucose and fructose; disaccharides such as maltose and sucrose; polysaccharides such as dextrin and cyclodextrin; and sugar alcohols such as xylitol, sorbitol and erythritol. Sweeteners may include natural sweeteners such as thaumatin and stevia extract; and synthetic sweeteners such as saccharin and aspartame. The proportion of the natural carbohydrates may generally be approximately 0.01 to 0.04 g, specifically, approximately 0.02 to 0.03 g per 100 mL of the health beverage composition of the present invention.

In addition to the above materials, the health beverage composition may contain various nutrients, vitamins, electrolytes, flavorings, coloring agents, pectic acid, salts of pectic acid, alginic acid, salts of alginic acid, organic acids, protective colloid thickeners, a pH regulator, a stabilizer, a preservative, glycerin, an alcohol, or a carbonating agent. Additionally, the health beverage composition may contain pulp for manufacturing a natural fruit juice, a fruit juice drink, or a vegetable drink. These ingredients may be used independently or in combination. The proportion of such additives is not very important, but is usually selected in the range of 0.01 to 0.1 part by weight per 100 parts by weight of the health beverage composition of the present invention.

Although the food composition of the present invention may be included at various wt% as long as it can exhibit an effect of preventing or improving hypomelanosis, the peptide of the present invention may be specifically included at 0.00001 to 100 wt% or 0.01 to 80 wt% with respect to the total weight of the food composition, but the present invention is not limited thereto.

### [Advantageous Effects]

A peptide or a composition including the same according to an embodiment of the present invention can promote tyrosinase activity involved in the formation of melanosomes in melanocytes.

The peptide or the composition including the same according to an embodiment of the present invention can regulate tyrosinase activity in melanocytes to prevent hypomelanosis which causes depigmented patches on the skin.

The peptide or the composition including the same according to an embodiment of the present invention can promote melanogenesis in melanocytes to improve or treat hypomelanosis.

The peptide or the composition including the same according to an embodiment of the present invention can promote the process of oxidizing L-DOPA to DOPA-quinone during melanin synthesis in melanocytes.

The effects of the present invention are not limited to the effects mentioned above, and other effects that have not been mentioned will be clearly understood by those of ordinary skill in the art from the description below.

### [Description of Drawings]

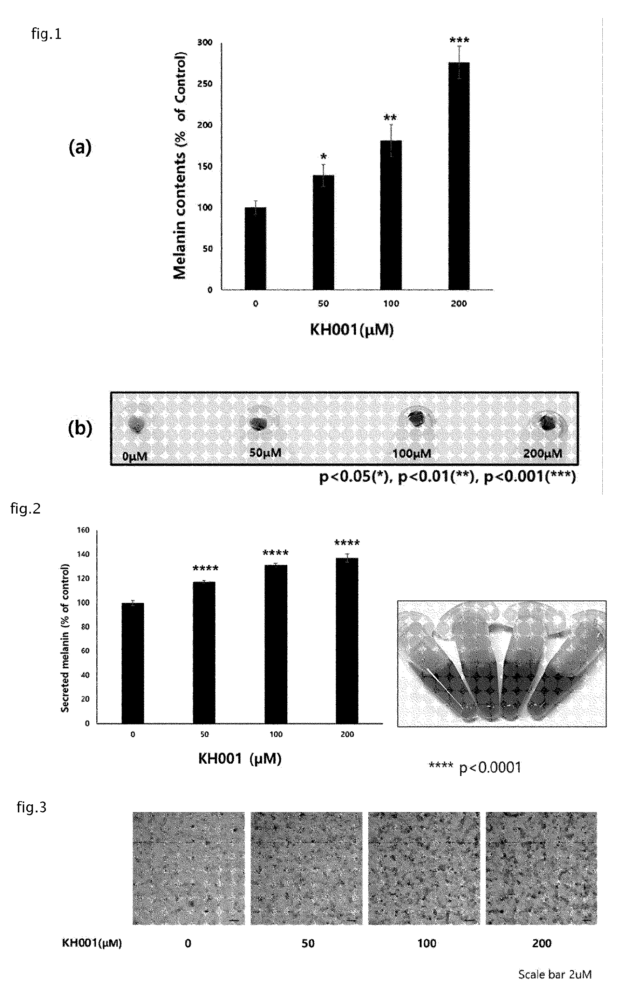

FIG. 1 shows the results of measuring an absorbance at 405 nm after B 16F10 cells are seeded at 230,000 cells/well in a 6-well plate, cultured at 37 °C in a 5% CO₂ incubator for 24 hours, and treated with different concentrations of peptide KH001 (SEQ ID NO: 96) for 72 hours to prepare cells, and then the cell pellet is dissolved in a strong base solution, the results showing (a) the melanin content (%) per peptide treatment concentration and (b) the degree of melanogenesis per peptide treatment concentration.
FIG. 2 shows tyrosinase activity after seeding B16F10 cells at 230,000 cells/well in a 6-well plate, culturing the cells at 37 °C in a 5% CO₂ incubator for 24 hours, treating the cells with different concentrations of peptide KH001 (SEQ ID NO: 96) for 72 hours to prepare cells, fixing the cells, and then observing a product obtained by reacting with L-DOPA using a light microscope.
FIG. 3 shows the value of tyrosinase activity (corrected for the protein concentration of the cell lysate) measured after seeding B16F10 cells at 230,000 cells/well in a 6-well plate, culturing the cells at 37 °C in a 5% CO₂ incubator for 24 hours, treating the cells with the KH001 peptide at different concentrations of 50 µM, 100 µM, and 200 µM for 72 hours, washing the cells with PBS, lysing all of the cells with 100 µL of 0.1M potassium phosphate buffer (pH 6.8) containing 1% Triton X, centrifuging the cells in a 1.5 mL tube at 12000 rpm for 20 minutes to obtain 40 µL of the supernatant, reacting the supernatant with 200 µL of 2 mg/ml L-DOPA solution (0.1M potassium phosphate, pH 7.0) at 37 °C for 30 minutes, and measuring an absorbance at 475 nm.

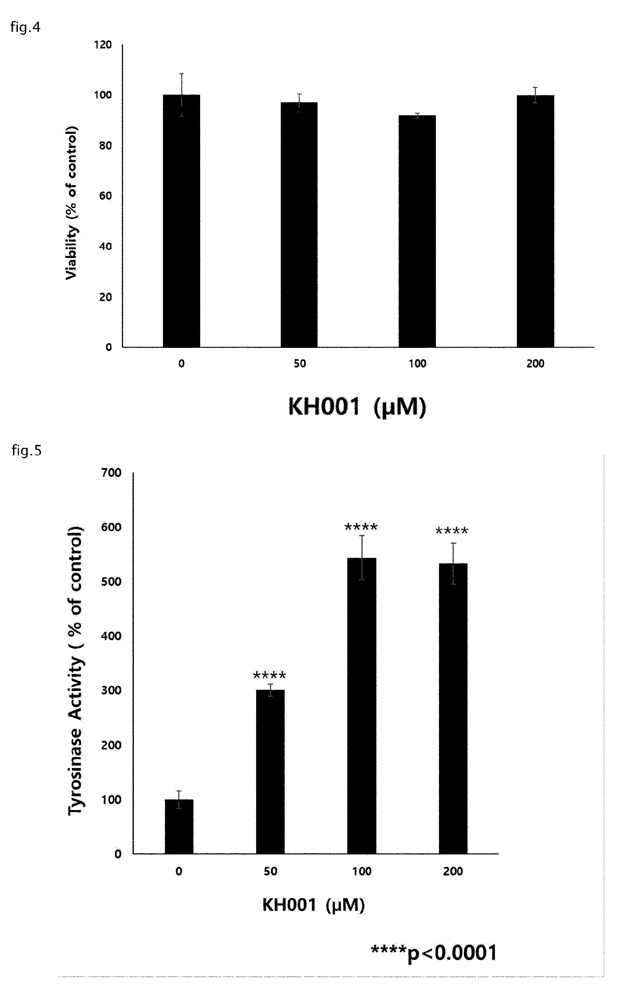

FIG. 4 shows the results of quantifying secreted melanin after seeding B16F10 cells at 230,000 cells/well in a 6-well plate, culturing the cells at 37 °C in a 5% CO₂ incubator for 24 hours, treating the cells with the KH001 peptide at different concentrations of 50 µM, 100 µM, and 200 µM, incubating the cells for 72 hours, and measuring an absorbance of the medium in each well at 405 nm.
FIG. 5 shows the results of measuring cell viability by MTT assay 72 hours after treating B16F10 cells with the KH001 peptide at a concentration of 50 µM, 100 µM, or 200 µM.

### [Modes of the Invention]

The purpose and effects of the present invention, and the technical configurations for achieving them will become clear with reference to the embodiments described in detail below together with the accompanying drawings. In explaining the present invention, when it is judged that a detailed description of a known function or configuration may unnecessarily obscure the gist of the present invention, the detailed description will be omitted. In addition, the terms described below are terms defined in consideration of the donation in the present invention, and these may vary depending on the intention or custom of a user or operator.

However, the present invention is not limited to the embodiments disclosed below and may be implemented in a variety of different forms. These embodiments are provided solely to make the disclosure of the present invention complete and to fully inform those skilled in the art of the scope of the present invention, and the present invention is defined only by the scope of the claims. Therefore, the definition should be based on the contents throughout this specification.

Hereinafter, embodiments of the present invention will be described in detail.

### Example 1: Synthesis of peptide

The present inventors synthesized a peptide (SEQ ID NO: 1) exhibiting a tyrosinase activity promoting effect by a 9-fluorenylmethyloxycarbonyl (Fmoc) method, and synthesized peptides of each group by substituting amino acids of the synthesized peptide (Tables 1 to 12).
N-KYQRRKKNKY-C (SEQ ID NO: 1)

First, the peptides of Group 1 were synthesized by synthesizing the peptide of SEQ ID NO: 1 or by substituting the amino acids at positions 5 to 7 of the peptide of SEQ ID NO: 1 with lysine or arginine (Table 1).

**[Table 1]**

| SEQ ID NO | Amino acid sequence (N-C) |
|---|---|
| 1 | KYQRRKKNKY |
| 2 | KYQRRKRNKY |
| 3 | KYQRRRKNKY |
| 4 | KYQRRRRNKY |
| 5 | KYQRKKKNKY |
| 6 | KYQRKRKNKY |
| 7 | KYQRKKRNKY |
| 8 | KYQRKRRNKY |

Next, the peptides of Group 2 were synthesized by substituting the amino acids at positions 5 to 7 of the peptide of SEQ ID NO: 1 with lysine or arginine and the amino acid at position 8 thereof with serine (Table 2).

**[Table 2]**

| SEQ ID NO | Amino acid sequence (N-C) |
|---|---|
| 9 | KYQRRKKSKY |
| 10 | KYQRRKRSKY |
| 11 | KYQRRRKSKY |
| 12 | KYQRRRRSKY |
| 13 | KYQRKKKSKY |
| 14 | KYQRKRKSKY |
| 15 | KYQRKKRSKY |
| 16 | KYQRKRRSKY |

Next, the peptides of Group 3 were synthesized by substituting the amino acids at positions 5 to 7 of the peptide of SEQ ID NO: 1 with lysine or arginine, the amino acid at position 9 thereof with tyrosine, and the amino acid at position 10 thereof with lysine (Table 3).

**[Table 3]**

| SEQ ID NO | Amino acid sequence (N-C) |
|---|---|
| 17 | KYQRRKKNYK |
| 18 | KYQRRKRNYK |
| 19 | KYQRRRKNYK |
| 20 | KYQRRRRNYK |
| 21 | KYQRKKKNYK |
| 22 | KYQRKRKNYK |
| 23 | KYQRKKRNYK |
| 24 | KYQRKRRNYK |

Next, the peptides of Group 4 were synthesized by substituting the amino acids at positions 5 to 7 of the peptide of SEQ ID NO: 1 with lysine or arginine, the amino acid at position 8 thereof with serine, the amino acid at position 9 thereof with tyrosine, and the amino acid at position 10 thereof with lysine (Table 4).

**[Table 4]**

| SEQ ID NO | Amino acid sequence (N-C) |
|---|---|
| 25 | KYQRRKKSYK |
| 26 | KYQRRKRSYK |
| 27 | KYQRRRKSYK |
| 28 | KYQRRRRSYK |
| 29 | KYQRKKKSYK |
| 30 | KYQRKRKSYK |
| 31 | KYQRKKRSYK |
| 32 | KYQRKRRSYK |

Next, the peptides of Group 5 were synthesized by substituting the amino acid at position 3 of the peptide of SEQ ID NO: 1 with arginine, the amino acid at position 4 thereof with glutamine, and the amino acids at positions 5 to 7 thereof with lysine or arginine (Table 5).

**[Table 5]**

| SEQ ID NO | Amino acid sequence (N-C) |
|---|---|
| 33 | KYRQRKKNKY |
| 34 | KYRQRKRNKY |
| 35 | KYRQRRKNKY |
| 36 | KYRQRRRNKY |
| 37 | KYRQKKKNKY |
| 38 | KYRQKRKNKY |
| 39 | KYRQKKRNKY |
| 40 | KYRQKRRNKY |

Next, the peptides of Group 6 were synthesized by substituting the amino acid at position 3 of the peptide of SEQ ID NO: 1 with arginine, the amino acid at position 4 thereof with glutamine, the amino acids at positions 5 to 7 thereof with lysine or arginine, and the amino acid at position 8 thereof with serine (Table 6).

**[Table 6]**

| Peptides of Group 6 | |
|---|---|
| SEQ ID NO | Amino acid sequence (N-C) |
| 41 | KYRQRKKSKY |
| 42 | KYRQRKRSKY |
| 43 | KYRQRRKSKY |
| 44 | KYRQRRRSKY |
| 45 | KYRQKKKSKY |
| 46 | KYRQKRKSKY |
| 47 | KYRQKKRSKY |
| 48 | KYRQKRRSKY |

Next, the peptides of Group 7 were synthesized by substituting the amino acid at position 3 of the peptide of SEQ ID NO: 1 with arginine, the amino acid at position 4 thereof with glutamine, the amino acids at positions 5 to 7 with lysine or arginine, the amino acid at position 9 thereof with tyrosine, and the amino acid at position 10 thereof with lysine (Table 7).

**[Table 7]**

| SEQ ID NO | Amino acid sequence (N-C) |
|---|---|
| 49 | KYRQRKKNYK |
| 50 | KYRQRKRNYK |
| 51 | KYRQRRKNYK |
| 52 | KYRQRRRNYK |
| 53 | KYRQKKKNYK |
| 54 | KYRQKRKNYK |
| 55 | KYRQKKRNYK |
| 56 | KYRQKRRNYK |

Next, the peptides of Group 8 were synthesized by substituting the amino acid at position 3 of the peptide of SEQ ID NO: 1 with arginine, the amino acid at position 4 thereof with glutamine, the amino acids at positions 5 to 7 with lysine or arginine, the amino acid at position 8 thereof with serine, the amino acid at position 9 thereof with tyrosine, and the amino acid at position 10 thereof with lysine (Table 8).

**[Table 8]**

| SEQ ID NO | Amino acid sequence (N-C) |
|---|---|
| 57 | KYRQRKKSYK |
| 58 | KYRQRKRSYK |
| 59 | KYRQRRKSYK |
| 60 | KYRQRRRSYK |
| 61 | KYRQKKKSYK |
| 62 | KYRQKRKSYK |
| 63 | KYRQKKRSYK |
| 64 | KYRQKRRSYK |

Next, the peptides of Group 9 were synthesized by substituting the amino acid at position 3 of the peptide of SEQ ID NO: 1 with lysine, the amino acid at position 4 thereof with glutamine, and the amino acids at positions 5 to 7 with lysine or arginine (Table 9).

**[Table 9]**

| SEQ ID NO | Amino acid sequence (N-C) |
|---|---|
| 65 | KYKQRKKNKY |
| 66 | KYKQRKRNKY |
| 67 | KYKQRRKNKY |
| 68 | KYKQRRRNKY |
| 69 | KYKQKKKNKY |
| 70 | KYKQKRKNKY |
| 71 | KYKQKKRNKY |
| 72 | KYKQKRRNKY |

Next, the peptides of Group 10 were synthesized by substituting the amino acid at position 3 of the peptide of SEQ ID NO: 1 with lysine, the amino acid at position 4 thereof with glutamine, the amino acids at positions 5 to 7 with lysine or arginine, and the amino acid at position 8 thereof with serine (Table 10).

**[Table 10]**

| SEQ ID NO | Amino acid sequence (N-C) |
|---|---|
| 73 | KYKQRKKSKY |
| 74 | KYKQRKRSKY |
| 75 | KYKQRRKSKY |
| 76 | KYKQRRRSKY |
| 77 | KYKQKKKSKY |
| 78 | KYKQKRKSKY |
| 79 | KYKQKKRSKY |
| 80 | KYKQKRRSKY |

Next, the peptides of Group 11 were synthesized by substituting the amino acid at position 3 of the peptide of SEQ ID NO: 1 with lysine, the amino acid at position 4 thereof with glutamine, the amino acids at positions 5 to 7 with lysine or arginine, the amino acid at position 9 thereof with tyrosine, and the amino acid at position 10 thereof with lysine (Table 11).

**[Table 11]**

| SEQ ID NO | Amino acid sequence (N-C) |
|---|---|
| 81 | KYKQRKKNYK |
| 82 | KYKQRKRNYK |
| 83 | KYKQRRKNYK |
| 84 | KYKQRRRNYK |
| 85 | KYKQKKKNYK |
| 86 | KYKQKRKNYK |
| 87 | KYKQKKRNYK |
| 88 | KYKQKRRNYK |

Finally, the peptides of Group 12 were synthesized by substituting the amino acid at position 3 of the peptide of SEQ ID NO: 1 with lysine, the amino acid at position 4 thereof with glutamine, the amino acids at positions 5 to 7 with lysine or arginine, the amino acid at position 8 thereof with serine, the amino acid at position 9 thereof with tyrosine, and the amino acid at position 10 thereof with lysine (Table 12).

**[Table 12]**

| SEQ ID NO | Amino acid sequence (N-C) |
|---|---|
| 89 | KYKQRKKSYK |
| 90 | KYKQRKRSYK |
| 91 | KYKQRRKSYK |
| 92 | KYKQRRRSYK |
| 93 | KYKQKKKSYK |
| 94 | KYKQKRKSYK |
| 95 | KYKQKKRSYK |
| 96 | KYKQKRRSYK |

### Example 2: Materials and methods

### Example 2-1. Culture of melanocytes

A melanocyte (B16F10) cell line was cultured using DMEM containing 10% FBS and 1% penicillin/streptomycin (PS) at 37 °C in a 5% CO₂ incubator.

### Example 2-2. MTT assay

An MTT reagent was used by being diluted in DMEM at a concentration of 0.5 mg/mL.

B16F10 cells were seeded at 230,000 cells/well in a 6-well plate, and cultured at 37 °C in a 5% CO₂ incubator for 24 hours. Subsequently, the cells were treated with peptide KH001 (SEQ ID NO: 96) at different concentrations for 72 hours to prepare the cells, and then the MTT reagent was added and allowed to react in a 37 °C incubator for 1 hour under light blocking conditions. After removing the entire medium, the generated formazan was dissolved in DMSO, and then absorbance was measured at 560 nm.

### Example 2-3. Quantification of melanin content

B16F10 cells were seeded at 230,000 cells/well in a 6-well plate, and cultured at 37 °C in a 5% CO₂ incubator for 24 hours. The cells were treated with the KH001 peptide at different concentrations of 50 µM, 100 µM, and 200 µM for 72 hours. The cells were washed with PBS, and lysed with a solution in which 10% DMSO was added to 1N NaOH. Absorbance was measured at 405 nm to quantify the amount of melanin in the cell lysate, and corrected for the protein concentration in the lysate. Melanin content (%) = (Absorbance of peptide-treated group / Absorbance of PBS-treated group) X 100

### Example 2-4. Quantification of secreted melanin

B16F10 cells were seeded at 230,000 cells/well in a 6-well plate, and cultured at 37 °C in a 5% CO₂ incubator for 24 hours. The cells were treated with the KH001 peptide at different concentrations of 50 µM, 100 µM, and 200 µM for 72 hours. Here, secreted melanin was quantified by measuring the absorbance of the medium in each well at 405 nm. Secreted melanin (%) = (Absorbance of peptide-treated group / Absorbance of PBS-treated group) X 100

### Example 2-5. Measurement of tyrosinase activity

B16F10 cells were seeded at 230,000 cells/well in a 6-well plate, and cultured at 37 °C in a 5% CO₂ incubator for 24 hours. The cells were treated with the KH001 peptide at different concentrations of 50 µM, 100 µM, and 200 µM for 72 hours. Subsequently, the cells were washed with PBS, lysed thoroughly with 100 µL of 0.1M potassium phosphate buffer (pH 6.8) containing 1% Triton X, put into a 1.5 mL tube, and centrifuged at 12000 rpm for 20 minutes. Here, 40 µL of the supernatant was taken and allowed to react with 200 µL of 2 mg/mL L-DOPA solution (0.1M potassium phosphate, pH 7.0) at 37 °C for 30 minutes. Subsequently, tyrosinase activity was measured by measuring an absorbance at 475 nm. This value was corrected for the protein concentration in the cell lysate. Tyrosinase activity (%) = (Absorbance of peptide-treated group / Absorbance of PBS-treated group) X 100

### Example 2-6. Measurement of tyrosinase activity 2

B16F10 cells were seeded at 230,000 cells/well in a 6-well plate, and cultured at 37 °C in a 5% CO₂ incubator for 24 hours. The cells were treated with the KH001 peptide at different concentrations of 50 µM, 100 µM, and 200 µM for 72 hours. After washing the cells with PBS, the cells were fixed with 2% para-formaldehyde for 20 minutes. Subsequently, the cells were washed three times with PBS, and then 0.1% L-DOPA (0.1M potassium phosphate, pH 7.0) was added to each well. Subsequently, the reaction was carried out in a 37 °C incubator for 3 hours, and then photographs were taken using an light microscope.

### Example 3: Results

### Example 3-1. Effect of peptide KH001 on melanogenesis in B16F10 melanocytes

B16F10 cells were treated with the KH001 peptide at different concentrations. After 72 hours of peptide treatment, the cell pellet was lysed with a strong base, absorbance was measured at 405 nm to quantify the amount of melanin in the cells, and the melanin amount was corrected for a protein level using a BSA assay. Referring to FIG. 1, as a result of treating the cells with KH001 at 50 µM, 100 µM, and 200 µM, melanogenesis increased by 38%, 81%, and 176%, respectively, compared to the control. Accordingly, it can be seen that the KH001 peptide induces melanin synthesis in concentration-dependent manner.

### Example 3-2. Effect of peptide KH001 on tyrosinase activity in B16F10 melanocytes 1

In the melanin synthesis process, L-DOPA is oxidized to DOPA-quinone, which is a process that requires the activity of tyrosinase. Accordingly, in this study, tyrosinase activity was measured by quantifying DOPA-quinone produced by reacting the lysate of B 16F10 cells treated with the KH001 peptide with L-DOPA. Referring to FIG. 5, compared to the control, tyrosinase activity increased by 101%, 443%, and 433% at KH001 treatment concentrations of 50 µM, 100 µM, and 200 µM, respectively. Accordingly, it can be seen that the KH001 peptide induces melanin synthesis by increasing tyrosinase activity.

### Example 3-3. Effect of peptide KH001 on tyrosinase activity of B16F10 melanocytes 2

B16F10 cells were treated with the KH001 peptide at different concentrations for 72 hours, fixed, and allowed to react with L-DOPA reagent. When tyrosinase in the cells reacts with L-DOPA, a black color appears, and the blacker the color, the higher the tyrosinase activity. Referring to FIG. 2, it can be seen that tyrosinase activity is significantly increased in the KH001 peptide-treated group compared to the control.

### Example 3-4. Effect of peptide KH001 on melanin secretion from B16F10 melanocytes

72 hours after B16F10 cells were treated with the KH001 peptide at different concentrations, the amount of melanin secreted into the culture medium was quantified by measuring an absorbance at 405 nm, and referring to FIG. 2, as a result of KH001 peptide treatment, melanogenesis increased by 17%, 31%, and 37% in a concentration-dependent manner compared to the control. Therefore, it can be seen that the B16F10 cells secrete more melanin in a concentration-dependent manner due to the effect of the KH001 peptide.

### Example 3-5. Evaluation of toxicity of peptide KH001 in B16F10 melanocytes

B16F10 cells were treated with the KH001 peptide at different concentrations. After 72 hours after KH001 peptide treatment, cell viability was measured using an MTT assay. Referring to FIG. 4, there was no significant difference in cell viability compared to the control when treated with KH001 at different concentrations of 50 µM, 100 µM, and 200 µM.

Therefore, it was confirmed that the KH001 peptide had no toxicity at the predetermined concentrations.

The exemplary embodiments of the present invention have been disclosed in the specification and drawings. Although specific terms are used, they are used only in a general sense to easily explain the technical contents of the present invention and to help understand the present invention, and are not intended to limit the scope of the present invention. In addition to the embodiments disclosed herein, it is apparent to those of ordinary skill in the art that other modifications based on the technical idea of the present invention are possible.

## Claims

1. A composition for promoting tyrosinase activity, comprising:
a peptide consisting of an amino acid sequence of General Formula 1 below:
K-Y-R1-R2-R3-R4-R5-R6-R7-R8 (General Formula 1),
wherein, in General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are each lysine (K) or tyrosine (Y).

2. A composition for regulating melanin synthesis, comprising a peptide consisting of an amino acid sequence of General Formula 1 below:
K-Y-R1-R2-R3-R4-R5-R6-R7-R8 (General Formula 1),
wherein, in General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are each lysine (K) or tyrosine (Y).

3. A composition for preventing, improving, or treating hypomelanosis, comprising a peptide consisting of an amino acid sequence of General Formula 1 below:
K-Y-R1-R2-R3-R4-R5-R6-R7-R8 (General Formula 1)
wherein, in General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are each lysine (K) or tyrosine (Y).

4. A quasi-drug composition for preventing or improving hypomelanosis, comprising a peptide consisting of an amino acid sequence of General Formula 1 below:
K-Y-R1-R2-R3-R4-R5-R6-R7-R8 (General Formula 1)
wherein, in General Formula 1,
R1 is arginine (R), lysine (K), or glutamine (Q);
R2 is arginine (R) or glutamine (Q);
R3, R4, and R5 are each arginine (R) or lysine (K);
R6 is asparagine (N) or serine (S); and
R7 and R8 are each lysine (K) or tyrosine (Y).

5. The composition of claim 1, wherein the peptide is represented by an amino acid sequence of any one of SEQ ID NOs: 1 to 96.

6. A polynucleotide encoding the peptide of claim 1.

7. An expression vector comprising the polynucleotide of claim 6.

8. The composition of claim 1, wherein the composition comprises a polypeptide in which the peptide is linked repeatedly.

9. The composition of claim 3, wherein the hypomelanosis is vitiligo or poliosis.

10. The composition of claim 3, wherein the composition further comprises a pharmaceutically acceptable carrier, excipient or diluent.

11. A method of preventing, improving or treating hypomelanosis by administering the composition of claim 3 to a subject.
